# EUROPEAN PATENT SPECIFICATION

(11) **EP 4 346 809 B1**
(45) Date of publication and mention of the grant of the patent: **15.07.2026**
(21) Application number: 22729797.5
(22) Date of filing: 25.05.2022
(51) Int. Cl.: A61K 31/423, A61K 31/7048, A61P 1/16

(54) **PEMAFIBRATE FOR USE IN TREATING LIVER DISEASE**
PEMAFIBRAT ZUR VERWENDUNG BEI DER BEHANDLUNG VON LEBERERKRANKUNGEN
PEMAFIBRATE POUR UTILISATION DANS LE TRAITEMENT D'UNE MALADIE HÉPATIQUE

(30) Priority: 27.05.2021 US 202163194088 P
(43) Date of publication of application: 10.04.2024
(60) Divisional application: 26183655.5
(73) Proprietor: KOWA COMPANY, LTD., Naka-ku Nagoya-shi Aichi 460-8625 (JP)
(72) Inventor: KANETA, Noboru, Nagoya-shi, Aichi 460-8625 (JP); OSHIMA, Ryu, Nagoya-shi, Aichi 460-8625 (JP); PENDSE, Shona Sanchita, Nagoya-shi, Aichi 460-8625 (JP); TANIGAWA, Ryohei, Nagoya-shi, Aichi 460-8625 (JP)
(74) Representative: Fuchs Patentanwälte Partnerschaft mbB
(86) International application number: PCT/IB2022/054867
(87) International publication number: WO 2022/249071

(56) References cited:
- US-A1- 2016 136 138
- RATZIU VLAD ET AL: "Why Do So Many Nonalcoholic Steatohepatitis Trials Fail?", GASTROENTEROLOGY, 1 July 2023 (2023-07-01), pages 5 - 10, XP093239350, Retrieved from the Internet <URL:https://www.sciencedirect.com/science/article/pii/S0016508520306806?via%3Dihub>
- HONDA YASUSHI ET AL: "Supplementary information: Pemafibrate, a novel selective peroxisome proliferator-activated receptor alpha modulator, improves the pathogenesis in a rodent model of nonalcoholic steatohepatitis", SCIENTIFIC REPORTS, vol. 7, no. 1, 14 February 2017 (2017-02-14), US, pages 1 - 8, XP093340407, ISSN: 2045-2322, Retrieved from the Internet <URL:https://static-content.springer.com/esm/art:10.1038/srep42477/MediaObjects/41598_2017_BFsrep42477_MOESM75_ESM.pdf> DOI: 10.1038/srep42477
- HONDA YASUSHI ET AL: "Pemafibrate, a novel selective peroxisome proliferator-activated receptor alpha modulator, improves the pathogenesis in a rodent model of nonalcoholic steatohepatitis", SCIENTIFIC REPORTS, vol. 7, no. 1, 14 February 2017 (2017-02-14), XP055952791, Retrieved from the Internet <URL:http://www.nature.com/articles/srep42477.pdf> DOI: 10.1038/srep42477
- SASAKI YUSUKE ET AL: "Pemafibrate, a selective PPAR[alpha] modulator, prevents non-alcoholic steatohepatitis development without reducing the hepatic triglyceride content", SCIENTIFIC REPORTS, vol. 10, no. 1, 8 May 2020 (2020-05-08), pages 1 - 10, XP055952772, Retrieved from the Internet <URL:http://www.nature.com/articles/s41598-020-64902-8> DOI: 10.1038/s41598-020-64902-8
- ANONYMOUS: "A Study of Pemafibrate in Patients With Nonalcoholic Fatty Liver Disease (NAFLD)", CLINICALTRIALS.GOV, 9 April 2021 (2021-04-09), pages 1 - 6, XP055952941, Retrieved from the Internet <URL:https://www.clinicaltrials.gov/ct2/show/NCT03350165?term=pemafibrate&cond=nash&draw=2&rank=1> [retrieved on 20220818]
- KOMATSU TOSHINORI ET AL: "Real-world Profile of a Selective Peroxisome Proliferator-activated Receptor [alpha] Modulator (SPPARM[alpha]) in Japanese Patients with Renal Impairment and Dyslipidemia", INTERNAL MEDICINE., vol. 60, no. 17, 29 March 2021 (2021-03-29), JP, pages 2741 - 2748, XP055952943, ISSN: 0918-2918, Retrieved from the Internet <URL:chrome-extension://efaidnbmnnnibpcajpcglclefindmkaj/https://www.jstage.jst.go.jp/article/internalmedicine/advpub/0/advpub_6871-20/_pdf/-char/en> DOI: 10.2169/internalmedicine.6871-20
- GOTO RYO ET AL: "Inhibition of sodium glucose cotransporter 2 (SGLT2) delays liver fibrosis in a medaka model of nonalcoholic steatohepatitis (NASH).", FEBS OPEN BIO, vol. 9, no. 4, 15 February 2019 (2019-02-15), US, pages 643 - 652, XP055952870, ISSN: 2211-5463, Retrieved from the Internet <URL:https://onlinelibrary.wiley.com/doi/full-xml/10.1002/2211-5463.12598> DOI: 10.1002/2211-5463.12598
- DAVID E KLEINER ET AL: "Design and validation of a histological scoring system for nonalcoholic fatty liver disease", HEPATOLOGY, JOHN WILEY & SONS, INC, US, vol. 41, no. 6, 24 May 2005 (2005-05-24), pages 1313 - 1321, XP071560541, ISSN: 0270-9139, DOI: 10.1002/HEP.20701
- NAKAJIMA ATSUSHI ET AL: "Randomised clinical trial: Pemafibrate, a novel selective peroxisome proliferator-activated receptor [alpha] modulator (SPPARM[alpha]), versus placebo in patients with non-alcoholic fatty liver disease", ALIMENTARY PHARMACOLOGY & THERAPEUTICS., vol. 54, no. 10, 16 September 2021 (2021-09-16), GB, pages 1263 - 1277, XP055952787, ISSN: 0269-2813, Retrieved from the Internet <URL:https://onlinelibrary.wiley.com/doi/full-xml/10.1111/apt.16596> DOI: 10.1111/apt.16596

## Description

### FIELD OF THE INVENTION

The present disclosure relates to pemafibrate, tofogliflozin, and combinations thereof for use in treating human patients with liver diseases, particularly NAFLD or NASH patients suffering from liver stiffness or liver fibrosis, NAFLD or NASH patients with elevated ALT levels, NAFLD or NASH patients with elevated LDL-C, and NAFLD or NASH patients with healthy triglyceride levels. The present invention relates to pemafibrate or a pharmaceutically acceptable salt thereof for use in a method of treating liver stiffness or liver fibrosis in a human patient with noncirrhotic NASH or NAFLD and a NASH CRN fibrosis score as defined in the claims.

### BACKGROUND OF THE INVENTION

Nonalcoholic fatty liver disease (NAFLD) is a condition in which excess fat builds up in the liver. NAFLD includes nonalcoholic fatty liver ("NAFL") where there is no evidence of hepatocellular injury, and nonalcoholic steatohepatitis ("NASH"), which is characterized by steatosis, inflammation, and ballooning. Most NAFLD develops as a result of obesity, diabetes mellitus, dyslipidemia, or hypertension. The numbers of patients with NAFLD and NASH are increasing worldwide owing to an increasingly obese population, and the prevalence is estimated to be 20-30% and 2-6%, respectively.

Pemafibrate is a selective PPARα modulator that is approved for the treatment of hyperlipidemia in Japan. Pemafibrate is described chemically as (R)-2-[3-[[N-(benzoxazol-2-yl)-N-3-(4-methoxyphenoxy)propyl]aminomethyl]phenoxy]butyric acid, and has the following chemical structure:

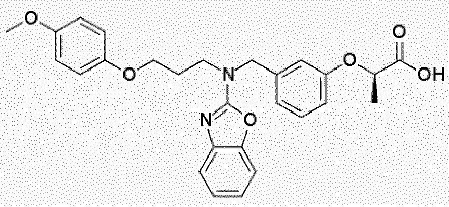

Pemafibrate regulates the expression of a gene cluster mainly related to lipid and sugar metabolism of the liver. It also increases gene expression for β-oxidation and lipid transport and enhances energy metabolism by the induction of mitochondrial uncoupling protein ("UCP") 3 gene expression. A non-clinical study in low density lipoprotein ("LDL") receptor knockout mice and KK-A mice fed with an MCD (methionine-choline-deficient) diet has shown that pemafibrate suppresses ballooning of hepatocytes and fat deposition and reduces the number of Kupffer cells. See US 2016/0136138 A1 to Shibata et al.

Anhydrous tofogliflozin is described chemically as 6-((4-ethylphenyl)methyl)-3',4',5',6'-tetrahydro-6'-(hydroxymethyl)spiro(isobenzofuran-1(3H),2'-(2H)pyran)-3',4',5'-triol, and has the following chemical structure: 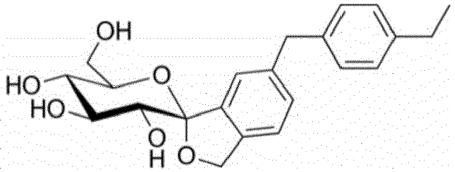 The United States Adopted Name ("USAN") tofogliflozin applies to the monohydrate, which is the form used as a drug. The International Nonproprietary Name ("INN") tofogliflozin applies to the anhydrous compound and the drug form is referred to as tofogliflozin hydrate. As used herein, tofogliflozin refers to the chemical compound 6-((4-ethylphenyl)methyl)-3',4',5',6'-tetrahydro-6'-(hydroxymethyl)spiro(isobenzofuran-1(3H),2'-(2H)pyran)-3',4',5'-triol, and thus includes all hydrated, solvated, crystalline, and amorphous forms thereof of the compound.

Tofogliflozin has previously been developed by Hoffman La-Roche Inc. and by Chugai Pharmaceutical Co, Ltd. for the treatment of type 2 diabetes mellitus (T2DM). Tofogliflozin is a potent and selective inhibitor of SGLT2, which is localized to the renal tubules and is responsible for reabsorption of glucose from the renal filtrate. A non-clinical study in an HHC mouse model of NASH has shown that a combination of pemafibrate and tofogliflozin reduces the size of lipid droplet in animal hepatocytes, with a resulting improvement in hepatocyte ballooning. See US 2020/0022960 A1 to Sasaki et al. Honda Yasushi et al. disclose in Scientific reports, 7(1), 2017, 1-11, that pemafibrate improves the pathogenesis of NASH in a rodent model.

There remains a need to treat and/or prevent liver stiffness and liver fibrosis in NASH and some NAFLD patients, in addition to the steatosis, inflammation, and ballooning that characterizes NASH and some NAFLD patients. There further remains a need to treat lipid and liver enzyme abnormalities in NAFLD and NASH patients, and to characterize patients that would benefit most from such treatment, particularly in terms of lipid profiles.

### SUMMARY OF INVENTION

The invention is as defined in the claims. The references to methods of treatment in this description are to be interpreted as references to the compounds, pharmaceutical compositions and medicaments of the present invention for use in a method for treatment of the human (or animal) body by therapy.

The inventors have unexpectedly discovered that pemafibrate, tofogliflozin, and combinations thereof, can treat and/or prevent liver stiffness and liver fibrosis in NASH and some NAFLD patients. Thus, in a first principal embodiment the invention provides pemafibrate or a pharmaceutically acceptable salt thereof for use in a method of treating liver stiffness or liver fibrosis in a human patient with noncirrhotic NASH or NAFLD and a NASH CRN fibrosis score of ≥ 1 and < 4 as defined in Table 3, the method comprising administering to the patient a therapeutically effective amount of pemafibrate or the pharmaceutically acceptable salt thereof. Optionally, the method may further comprise administering to the patient a therapeutically effective amount of tofogliflozin or a pharmaceutically acceptable salt thereof.

The inventors have further discovered that pemafibrate, tofogliflozin, and combinations thereof can treat NASH histology in NASH and NAFLD patients, particularly lobular inflammation. Thus, also disclosed is a method of treating lobular inflammation in a patient with noncirrhotic NASH or NAFLD comprising administering to the patient a therapeutically effective amount of: (a) pemafibrate or a pharmaceutically acceptable salt thereof; (b) tofogliflozin or a pharmaceutically acceptable salt thereof; or (c) a combination thereof. In a particularly preferred embodiment, the method is undertaken without worsening of fibrosis in a patient having a NASH CRN fibrosis score of ≥ 1 and < 4.

The inventors have further discovered that the treatments described herein result in unexpected improvements in liver enzyme activity, particularly in patients with fibrosis and high alanine aminotransferase ("ALT") levels. Thus, also disclosed is a method of improving liver function as measured by ALT activity in a patient with noncirrhotic NASH or NAFLD, a NASH CRN fibrosis score of ≥ 1 and < 4, and an ALT score ≥ 2X ULN, comprising administering to the patient a therapeutically effective amount of: (a) pemafibrate or a pharmaceutically acceptable salt thereof; (b) tofogliflozin or a pharmaceutically acceptable salt thereof; or (c) a combination thereof.

The inventors have also discovered that the treatments described herein result in unexpected improvements in LDL-C, particularly in patients with fibrosis and high LDL-C levels. also disclosed is a method of lowering LDL-C in a patient with noncirrhotic NASH or NAFLD, a NASH CRN fibrosis score of ≥ 1 and < 4, and an LDL-C concentration ≥ 100 mg/dL comprising administering to the patient a therapeutically effective amount of: (a) pemafibrate or a pharmaceutically acceptable salt thereof; (b) tofogliflozin or a pharmaceutically acceptable salt thereof; or (c) a combination thereof.

Surprisingly, any of the foregoing methods, including those that rely on pemafibrate, can be practiced in patients with normal triglyceride levels.

Additional advantages of the invention are set forth in part in the description which follows, and in part will be obvious from the description or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION

### Definitions and Use of Terms

As used in the specification and claims, the singular forms a, an, and the include plural references unless the context clearly dictates otherwise. For example, the term "a specification" refers to one or more specifications for use in the presently disclosed methods and systems. "An ingredient" includes mixtures of two or more such ingredients, and the like. The word "or" or like terms as used herein means any one member of a particular list and also includes any combination of members of that list.

As used in this specification and in the claims which follow, the word "comprise" and variations of the word, such as "comprising" and "comprises," means "including but not limited to," and is not intended to exclude, for example, other additives, components, integers or steps. When an element is described as comprising one or a plurality of components, steps or conditions, it will be understood that the element can also be described as "consisting of" or "consisting essentially of" the component, step or condition, or the plurality of components, steps or conditions.

"Therapeutically effective amount" means that amount which, when administered to a human for supporting or affecting a metabolic process, or for treating or preventing a disease, is sufficient to cause such treatment or prevention of the disease or supporting or affecting the metabolic process.

When "drug therapy," "drug administration," and like terms are used herein, it will be understood that the therapy can be accomplished through any suitable route of administration using any acceptable dosage form, and that the drug can be administered as the free base or acid, a salt, or an ester or other prodrug moiety.

When combination drug therapy is referred to herein for the achievement of a single endpoint or therapeutic objective, it will be understood that each of the individual active ingredients in the combination contributes to the achievement of the recited endpoint or therapeutic objective. Furthermore, it will be understood in preferred embodiments that each of the active ingredients contributes to a statistically significant clinical benefit in a suitably powered population of patients. When plural endpoints or therapeutic objectives are to be achieved, it will be understood that both of the individual active ingredients will contribute to the achievement of at least one of the endpoints or therapeutic objectives, preferably all of the endpoints and objectives, preferably to a statistically significant clinical benefit in a suitable powered population of patients, and any endpoints or therapeutic objectives not jointly contributed to will be achieved by at least one of the active ingredients, preferably to a statistically significant clinical benefit.

When used herein the term "about" will compensate for variability allowed for in the pharmaceutical industry and inherent in products in this industry, such as differences in product strength due to manufacturing variation and time-induced product degradation, as well as differences due to waters of hydration and different salts. The term allows for any variation which in the practice of good manufacturing practices, would allow the product being evaluated to be considered therapeutically equivalent or bioequivalent in humans to the recited strength of a claimed product. In one embodiment the term allows for any variation within 5% of the recited specification or standard. In one embodiment the term allows for any variation within 10% of the recited specification or standard.

When published test methodologies and diagnostic instruments are referred to herein, it will be understood that the test methodology or diagnostic instrument is performed based on the version in effect on May 1, 2021, unless otherwise stated to the contrary herein.

When ranges are expressed herein by specifying alternative upper and lower limits of the range, it will be understood that the endpoints can be combined in any manner that is mathematically feasible. Thus, for example, a range of from 50 or 80 to 100 or 70 can alternatively be expressed as a series of ranges of from 50 to 100, from 50 to 70, and from 80 to 100. When a series of upper bounds and lower bounds are related using the phase "and" or "or", it will be understood that the upper bounds can be unlimited by the lower bounds or combined with the lower bounds, and vice versa. Thus, for example, a range of greater than 40% and/or less than 80% includes ranges of greater than 40%, less than 80%, and greater than 40% but less than 80%. Unless otherwise specified by the term "between," the boundaries of the range (lower and upper ends of the range) are included in the claimed range.

In the context of the present invention insofar as it relates to any of the disease conditions recited herein, the term "treatment" means to reduce the occurrence of a symptom or condition, or to relieve or alleviate at least one symptom associated with such condition, or to slow or reverse the progression of such condition, or to manage or affect the metabolic processes underlying such condition. Within the meaning of the present invention, the term also denotes to arrest, or to "prevent," i.e. to delay the onset (i.e., the period prior to clinical manifestation of a disease) and/or reduce the risk of developing or worsening a disease. When a person with the condition is affirmatively recited, the term will be understood to require relief or alleviation of at least one symptom associated with the condition.

Biomarker test assays -- unless otherwise indicated herein, all biomarker test assays referred to herein are performed in accordance with standard procedures employed during the 2001-2002 cycle of the National Health and Nutrition Examination Survey.

### Discussion of Principal Embodiments

In a first principal embodiment the invention provides pemafibrate or a pharmaceutically acceptable salt thereof for use in a method of treating liver stiffness or liver fibrosis in a human patient with noncirrhotic NASH or NAFLD and a NASH CRN fibrosis score of ≥ 1 and < 4 as defined in Table 3, the method comprising administering to the patient a therapeutically effective amount of pemafibrate or the pharmaceutically acceptable salt thereof. Optionally, the method further comprises administering to the patient a therapeutically effective amount of tofogliflozin or a pharmaceutically acceptable salt thereof.

Also disclosed is a method of treating lobular inflammation in a patient with noncirrhotic NASH or NAFLD comprising administering to the patient a therapeutically effective amount of: (a) pemafibrate or a pharmaceutically acceptable salt thereof; (b) tofogliflozin or a pharmaceutically acceptable salt thereof; or (c) a combination thereof. In a particularly preferred embodiment, the method is undertaken without worsening of fibrosis, in a patient having a NASH CRN fibrosis score of ≥ 1 and < 4.

Also disclosed is a method of improving liver function as measured by ALT activity in a patient with noncirrhotic NASH or NAFLD, a NASH CRN fibrosis score of ≥ 1 and < 4, and an ALT score ≥ 2X ULN, comprising administering to the patient a therapeutically effective amount of: (a) pemafibrate or a pharmaceutically acceptable salt thereof; (b) tofogliflozin or a pharmaceutically acceptable salt thereof; or (c) a combination thereof.

Also disclosed is a method of lowering LDL-C in a patient with noncirrhotic NASH or NAFLD, a NASH CRN fibrosis score of ≥ 1 and < 4, and an LDL-C concentration ≥ 100 mg/dL, comprising administering to the patient a therapeutically effective amount of: (a) pemafibrate or a pharmaceutically acceptable salt thereof; (b) tofogliflozin or a pharmaceutically acceptable salt thereof; or (c) a combination thereof.

### Discussion of Subembodiments

The invention can further be understood with reference to various subembodiments which can modify any of the principal embodiments. It will be understood that these subembodiments can be combined in any manner that is both mathematically and physically possible to create additional subembodiments, which in turn can modify any of the principal embodiments.

As noted in the principal embodiments, the methods can be practiced using either pemafibrate individually, or pemafibrate and tofogliflozin in combination. Thus, in one subembodiment the methods are practiced by administering to the patient a therapeutically effective amount of pemafibrate or a pharmaceutically acceptable salt thereof. In another subembodiment the methods are practiced by administering to the patient a therapeutically effective amount of the combination of pemafibrate and tofoglifozin.

A preferred therapeutically effective amount of pemafibrate when orally administered, whether individually or in combination, is from 0.1 to 0.8 mg/day, or from 0.2 to 0.4 mg/day, in one, two or three divided doses. A particularly preferred dose is 0.4 mg/day. A preferred therapeutically effective amount of tofogliflozin when orally administered, whether individually or in combination is from 5 to 60 mg/day, or from 10 to 40 mg/day. A particularly preferred dose is 20 mg/day of tofogliflozin or a pharmaceutically acceptable salt thereof. The preferred dosing regimen is a single dosage form, preferably tablet, administered once daily. Thus, in a particularly preferred subembodiment the invention provides an orally administered unit dosage form comprising about 0.4 mg of pemafibrate or a pharmaceutically acceptable salt thereof and about 20 mg of tofogliflozin or a pharmaceutically acceptable salt thereof.

The patient treatable by the methods of the current invention can be characterized by several diagnostic criteria. Thus, in one subembodiment, at the time of commencing the method, the patient has a vibration-controlled transient elastography CAP score ≥ 290 dB/m. In another subembodiment, at the time of commencing the method, the patient has a LSM ≥ 7 kilopascals (kPa) and < 19 kPa. In another subembodiment, at the time of commencing the method, the patient has an AST concentration ≥ 20 U/L, 30 U/L, 40 U/L, or 50 U/L. In still another subembodiment, at the time of commencing the method, the patient has: (a) a vibration-controlled transient elastography CAP score ≥ 290 dB/m; (b) a LSM ≥ 7 kilopascals (kPa) and < 19 kPa; and (c) an AST concentration ≥ 20 U/L.

The patient can also be characterized histologically. According to the invention, at the time of commencing the method, the patient has a NASH CRN fibrosis score of ≥ 1 and < 4 as defined in Table 3.

In another subembodiment, at the time of commencing the method, the patient has a NAFLD activity score ("NAS") ≥ 4, ≥ 5, ≥ 6, ≥ 7, or = 8.

In another subembodiment, at the time of commencing the method, the patient has a NASH CRN steatosis score ≥ 1, ≥ 2, or = 3. In another subembodiment, at the time of commencing the method, the patient has a NASH CRN lobular inflammation score ≥ 1, ≥ 2, or = 3. In another subembodiment, at the time of commencing the method, the patient has a NASH CRN ballooning score ≥ 1 or = 2. In still another subembodiment, at the time of commencing the method, the patient has: (a) a NAS ≥ 4; (b) a NASH CRN steatosis score ≥ 1; (c) a NASH CRN lobular inflammation score ≥ 1; and (d) a NASH CRN ballooning score ≥ 1.

The patient can also be characterized histologically in addition to enzyme liver activity. Thus, in yet another subembodiment, at the time of commencing the method, the patient has: (a) a NAS ≥ 4; (b) a NASH CRN steatosis score ≥ 1; (c) a NASH CRN lobular inflammation score ≥ 1; (d) a NASH CRN ballooning score ≥ 1; and (e) an ALT concentration ≥ 1X, 2X, or 3X of ULN.

In other subembodiment the patient can be characterized by liver imaging and/or liver enzyme activity. Thus, in another subembodiment, at the time of commencing the method, the patient has a hepatic fat fraction on MRI-PDFF ≥ 10%, 12.5%, 15%, or 17.5%. In another subembodiment, at the time of commencing the method, the patient has liver stiffness on MRE ≥ 2.5 kPa, 2.75 kPa, or 3 kPa. In another subembodiment, at the time of commencing the method, the patient has an ALT concentration ≥ 1X, 2X, or 3X of ULN. In still another subembodiment, at the time of commencing the method, the patient has: (a) a hepatic fat fraction on MRI-PDFF ≥ 10%; (b) liver stiffness on MRE ≥ 2.5 kPa; and (c) an ALT level > ULN.

Still further criteria can be used to characterize the patients treatable by the methods of the current invention. Thus, in one subembodiment, at the time of commencing the method, the patient has an LDL-C concentration greater than 110 or 120 mg/dL.

In another subembodiment, at the time of commencing the method, the patient has a BMI greater than 22, 28, or 32 kg/m².

In another subembodiment, at the time of commencing the method, the patient has a fasting plasma glucose concentration ≥ 100 mg/dL.

In another subembodiment, at the time of commencing the method, the patient has a fasting TG level < 200, 175, or 150 mg/dL.

In still another subembodiment, at the time of commencing the method, the patient has a NASH CRN fibrosis score of 1, 2, 3, > 1 and < 4, or ≥ 2 and < 4.

In yet another subembodiment, at the time of commencing the method, the patient has an ALT concentration ≤ 5 × ULN.

Several different measures can be used to determine successful treatment according to the methods of the current invention. Thus, in one subembodiment the method comprises (a) a histological improvement in the patient's NAS of ≥ 2 points; and (b) no worsening of the patient's NASH CRN fibrosis score.

In another subembodiment the method comprises (a) histological resolution of steatohepatitis defined as: (i) absence of fatty liver disease; or (ii) isolated or simple steatosis without steatohepatitis and a NAS of 0-1 for inflammation, 0 for ballooning, and any value for steatosis; and (b) no worsening of NASH CRN fibrosis score.

In another subembodiment the method comprises (a) improvement in the patient's NASH CRN inflammation score ≥ 1; (b) improvement in the patient's NASH CRN ballooning score ≥ 1; and (c) improvement in the patient's NASH CRN steatosis score ≥ 1.

In another subembodiment the method comprises (a) improvement in the patient's NASH CRN fibrosis score ≥ 1; (b) no worsening of the patient's NASH CRN ballooning score; (c) no worsening of the patient's NASH CRN inflammation score; and (d) no worsening of the patient's NASH CRN steatosis score.

In still another subembodiment the method comprises (a) a liver fat content decrease as measured by MRI-PDFF of ≥ 30%; and/or (b) a ≥ 30% decrease in ALT to ≤ 40 U/L.

### EXAMPLES

In the following examples, efforts have been made to ensure accuracy with respect to numbers (e.g., amounts, temperature, etc.) but some errors and deviations should be accounted for. The following examples are put forth so as to provide those of ordinary skill in the art with a complete disclosure and description of how the methods claimed herein are made and evaluated, and are intended to be purely exemplary of the invention and are not intended to limit the scope of what the inventors regard as their invention, which is as defined in the claims.

### Example 1. Improvement of liver stiffness and serum markers of liver and lipid metabolism in patients with NAFLD.

This was a placebo-controlled, randomized, double-blind, parallel-group, study in adults with NAFLD. Subjects were administered pemafibrate tablets (0.4 mg/day, BID) or placebo tablets for 72 weeks following a screening period of from 2 to 8 weeks. The primary endpoint was percent change from baseline in liver fat content, measured by MRI-PDFF at week 24. The key secondary endpoints were percent changes in liver stiffness and ALT levels from baseline to week 72 and 24, respectively. The study was conducted in compliance with relevant guidelines, GCP guidance, and the Declaration of Helsinki.

Patients with a hepatic fat fraction ≥ 10% on MRI-PDFF, liver stiffness (measured using magnetic resonance elastography, MRE) ≥ 2.5 kPa and elevated ALT (>40 U/L for men, >30 U/L for women) were enrolled. Patients were excluded in case of body mass index (BMI) < 22 kg/m², uncontrolled diabetes (HbA1c ≥ 8%), impaired renal function (estimated glomerular filtration rate, eGFR, <30 mL • min⁻¹ • 1.73⁻¹ • m⁻² or on dialysis), cirrhosis, biliary obstruction, or chronic liver diseases other than NAFLD.

Subjects took their assigned medication (pemafibrate 0.2 mg tablet or placebo) orally, one tablet, twice daily, for 72 weeks. Study visits were set every 4 weeks from the start of treatment to week 24 and every 8 weeks from week 24 onwards. Liver fat content and liver stiffness were measured at screening examinations that took place pre-randomization and subsequently at weeks 0, 24, 48, and 72.

All imaging assessment procedures were performed blinded. The equipment used in all facilities was standardized to a 3.0T MR Imaging System (GE Healthcare, Little Chalfont, UK). As an application, IDEAL-IQ for MRI-PDFF and MR-touch for MRE were used. Detailed imaging conditions are described in the "Imaging Procedure Manual" and imaging procedures were standardized throughout all tests. Imaging was performed during fasting ( ≥ 4 h postprandial) and the timing of testing (pre-breakfast/post-breakfast to pre-lunch/post-lunch) was unified for each patient throughout the study.

The primary efficacy endpoint was percent change in hepatic fat content, as measured using MRI-PDFF. Liver stiffness measured using MRE was a secondary endpoint and similarly assessed percent change from baseline values. Other efficacy endpoints included liver function, fibrosis and inflammatory markers, and lipid markers.

Selects baseline characteristics of the study population are presented in Table 1. Select results are reported in Table 2.

**Table 1. baseline Characteristics of the Study Population (FAS)**

| | Placebo(n=60) | 0.4 mg pemafibrate (n=58) |
|---|---|---|
| **Demographics** | | |
| Age, years | 53.3(16.6) | 53.2(12.5) |
| Male | 37(61.7) | 31(53.4) |

| **Comorbidities** | | |
|---|---|---|
| Type2 Diabetes | 25(41.7) | 18(31.0) |
| Hyperlipidemia | 37(61.7) | 31(53.4) |
| Hypertension | 28(46.7) | 26(44.8) |
| Metabolic syndrome | 41(68.3) | 38(65.5) |

| **Liver image** | | |
|---|---|---|
| Liver fat content using MRI-PDFF, % | 18.05(5.48) | 18.75(6.89) |
| Liver stiffness using MRE , kPa | 3.02(0.44) | 3.24(0.81) |
| LSM stage using MRE | | |
| ≦1 | 30(50.0) | 26(44.8) |
| 2 | 25(41.7) | 21(36.2) |
| 3≦ | 5(8.3) | 11(19.0) |

| **Metabolic factors** | | |
|---|---|---|
| Weight, kg | 81.994(24.832) | 80.008(16.773) |
| Body Mass Index, kg/m² | 29.840(6.495) | 29.549(4.872) |
| Waist Circumference, cm | 99.914(15.093) | 100.211(10.402) |
| Fasting glucose, mg/dL | 111.3(17.9) | 109.7(17.3) |
| Hemoglobin A1C, % | 6.130(0.684) | 6.087(0.617) |

| **Liver function** | | |
|---|---|---|
| ALT, U/L | 94.58(49.38) | 82.83(36.63) |
| AST, U/L | 57.31(26.50) | 54.22(20.66) |

| **Lipids** | | |
|---|---|---|
| Total cholesterol, mg/dL | 202.08(36.81) | 209.34(33.87) |
| LDL cholesterol, mg/dL | 122.17(28.73) | 131.12(28.84) |
| HDL cholesterol, mg/dL | 48.44(11.32) | 48.96(8.86) |
| Non-HDL cholesterol, mg/dL | 153.64(36.41) | 160.38(30.75) |
| Triglyceride, mg/dL | 189.89(147.82) | 166.01(62.84) |

| | | |
|---|---|---|
| Data are Mean (SD) or n (%). | | |

| Table 2. Percent change from baseline in biomarkers | | | | | | | |
|---|---|---|---|---|---|---|---|
| | | Placebo (n=60) | | | Pemafibrate (n=58) | | |
| | | Week 24 | Week 48 | Week 72 | Week 24 | Week 48 | Week 72 |
| **Liver image** | | | | | | | |
| Liver fat content by MRI-PDFF | | -4.24 (-11.55,3.07) | -0.53 (-7.53,6.47) | 0.22 (-7.23,7.67) | -5.28 (-12.78,2.22) | 0.50 (-6.68,7.68) | -4.88 (-12.53,2.77) |
| | Mean difference | | | | -1.04 (-11.52,9.45) | 1.03 (-9.01,11.06) | -5.10 (-15.78,5.59) |
| | p value + | | | | 0.845 | 0.840 | 0.347 |
| Responder, % (n) (95%CI) | | 18.3 (11) (9.5,30.4) | 11.7(7) (4.8,22.6) | 11.7(7) (4.8,22.6) | 20.7 (12) (11.2,33.4) | 10.3 (6) (3.9,21.2) | 22.4 (13) (12.5,35.3) |
| | p value ‡ | | | | 0.818 | 1.000 | 0.145 |
| Liver stiffness by MRE | | -0.66 (-4.04,2.73) | -3.34 (-7.01,0.34) | -1.12 (-4.81,2.57) | -5.03 (-8.50,-1.56) | -9.01 (-12.79,-5.24) | -7.27 (-11.06,-3.48) |
| | Mean difference | | | | -4.38 (-9.26,0.51) | -5.68 (-10.98,-0.38) | -6.15 (-11.47,-0.83) |
| | p value ⁺ | | | | 0.078 | 0.036* | 0.024* |
| Responder, % (n) (95%Cl) | | 15.0 (9) (7.1,26.6) | 18.3 (11) (9.5,30.4) | 15.0 (9) (7.1,26.6) | 19.0(11) (9.9,31.4) | 37.9 (22) (25.5,51.6) | 25.9 (15) (15.3,39.0) |
| | p value ‡ | | | | *0.629* | 0.024* | 0.173 |

| **Liver function** | | | | | | | |
|---|---|---|---|---|---|---|---|
| ALT | | -12.46 (-20.83,-4.09) | -15.78 (-24.30,-7.26) | -10.24 (-19.18,-1.30) | -39.53 (-48.06,-31.01) | -42.53 (-51.29,-33.78) | -43.84 (-53.15,-34.53) |
| | Mean difference | | | | -27.08 (-39.05,-15.10) | -26.75 (-38.99,-14.52) | -33.60 (-46.53,-20.67) |
| | pvalue † | | | | <0.001** | <0.001** | <0.001** |
| AST | | -6.52 (-17.68,4.64) | -9.57 (-20.12,0.98) | -5.13 (-16.55,6.29) | -9.21 (-20.55,2.13) | -9.75 (-20.61,1.11) | -9.61 (-21.42,2.21) |
| | Mean difference | | | | -2.69 (-18.61,13.24) | -0.18 (-15.32,14.97) | -4.48 (-20.91,11.96) |
| | p value † | | | | 0.739 | 0.981 | 0.590 |

| **Lipids** | | | | | | | |
|---|---|---|---|---|---|---|---|
| Total cholesterol | | 0.36 (-2.64,3.36) | 1.64 (-1.11,4.39) | 3.74 (0.78,6.71) | -11.19 (-14.24,-8.14) | -11.55 (-14.37,-8.73) | -11.12 (-14.18,-8.06) |
| | Mean difference | | | | -11.55 (-15.83,-727) | -13.19 (-17.13,-9.24) | -14.86 (-19.13,-10.60) |
| | p value † | | | | <0.001** | <0.001** | <0.001** |
| LDL cholesterol | | -1.06 (-5.58,3.47) | 2.15 (-1.91,6.21) | 2.65 (-1.56,6.87) | -14.72 (-19.32,-10.11) | -14.22 (-18.39,-10.05) | -12.48 (-16.84,-8.13) |
| | Mean difference | | | | -13.66 (-20.14,-7.18) | -16.37 (-22.21,-10.52) | -15.14 (-21.22,-9.05) |
| | p value † | | | | <0.001** | <0.001** | <0.001** |
| HDL cholesterol | | 5.89 (2.31,9.46) | 3.84 (0.71,6.98) | 4.48 (1.03,7.93) | -1.68 (-5.32,1.96) | -6.40 (-9.61,-3.18) | -7.38 (-10.94,-3.82) |
| | Mean difference | | | | -7.57 (-12.67,-2.46) | -10.24 (-14.74,-5.74) | -11.86 (-16.82,-6.90) |
| | p value † | | | | 0.004** | <0.001** | <0.001** |
| Non-HDL cholesterol | | -1.00 (-4.60,2.59) | 1.28 (-2.08,4.64) | 3.85 (0.23,7.47) | -13.82 (-17.48,-10.16) | -12.95 (-16.40,-9.50) | -11.92 (-15.67,-8.18) |
| | | | | | 7.68) | 9.41) | 10.56) |
| | p value † | | | | <0.001** | <0.001** | <0.001** |
| Triglycerides | | -3.49 (-9.97,2.98) | -1.35 (-8.57,5.88) | 1.13 (-5.03,7.28) | -34.60 (-41.20,-28.01) | -27.11 (-34.56,-19.67) | -31.55 (-37.97,-25.13) |
| | Mean difference | | | | -31.11 (-40.36,-21.86) | -25.77 (-36.15,-15.39) | -32.67 (-41.58,-23.77) |
| | p value † | | | | <0.001** | <0.001** | <0.001** |

The significant improvements in liver stiffness when measured by MRE, corresponding to an improvement in liver fibrosis, was a significant surprising finding from this study. Other surprising findings from the study, particularly when compared to the results reported by S. Ishibashi et al. / Atherosclerosis 249 (2016) 36-43, include:
- The significant improvements in liver function as measured by ALT (Ishibashi reported a much more modest decrease in ALT concentrations from the administration of pemafibrate);
- The significant reductions in total cholesterol and LDL cholesterol (Ishibashi reported a modest decrease in total cholesterol and an increase in LDL cholesterol from the administration of pemafibrate); and
- The significant reductions in HDL cholesterol, particularly considering the HDL cholesterol and particle size data reported by Ishibashi 2016.

The improvements in liver stiffness, liver function, and cholesterol modification in spite of the reductions in HDL cholesterol could not have been predicted before this study.

### Example 2. A Multicenter, Placebo-Controlled, Randomized, Double-Blind, 48-Week Study Evaluating Pemafibrate, Tofoglifozin, and the Combination of Pemafibrate and Tofoglifozin in Patients with NASH and Liver Fibrosis

This is a double-blind, randomized, 48-week study assessing the efficacy and safety of pemafibrate monotherapy (0.4 mg/day), tofoglifozin monotherapy (20 mg/day), and K-001 (combination therapy of pemafibrate 0.4 mg/day and tofoglifozin 20 mg/day), compared with placebo, in patients with noncirrhotic NASH with liver fibrosis. Tofoglifozin monotherapy is for reference.

Probable patients with noncirrhotic NASH with liver fibrosis will first be identified using a FibroScan vibration-controlled transient elastography controlled attenuation parameter score ≥ 290 dB/m, FibroScan liver stiffness measurement ≥ 7 kilopascals (kPa) and < 19 kPa, and aspartate aminotransferase (AST) ≥ 20 U/L. Probable NASH patients will receive confirmatory liver biopsy for eligibility which will also be used as a baseline assessment for the histological assessments. Significant inclusion criteria are (i) NAS ≥ 4 with a score of at least 1 in each component of the NAS (steatosis, lobular inflammation, and ballooning) (ii) fibrosis stage of 1 or greater and below 4 on NASH CRN fibrosis staging system, and (iii) fasting plasma glucose ≥ 100 mg/dL.

The primary objective of this study is to evaluate efficacy in patients with noncirrhotic NASH with liver fibrosis. Efficacy determination will be based upon liver histological improvement in NAFLD activity score (NAS) ≥ 2 points and no worsening of fibrosis score at Week 48 as the primary endpoint.

The primary efficacy endpoint is improvement from baseline in disease activity and no worsening of liver fibrosis on the NASH Clinical Research Network (CRN) fibrosis score at Week 48. The improvement in disease activity is defined as improvement in NAS ≥ 2 points. The worsening of fibrosis is defined as any numerical increase in the stage.

The secondary efficacy endpoints of this study include:
- Resolution of steatohepatitis on overall histopathological reading and no worsening of liver fibrosis on NASH CRN fibrosis score at Week 48. Resolution of steatohepatitis is defined as absent fatty liver disease or isolated or simple steatosis without steatohepatitis and a NAS score of 0-1 for inflammation, 0 for ballooning, and any value for steatosis.
- Improvement from baseline in each of the NAS components (inflammation, ballooning, and steatosis) ≥ 1 point at Week 48
- Improvement from baseline in liver fibrosis score ≥ 1 and no worsening of steatohepatitis (defined as no increase in NAS for ballooning, inflammation, or steatosis) at Week 48
- Either improvement from baseline in liver fat content (MRI-PDFF) or improvement from baseline in ALT at Week 48. Improvement in liver fat content, or improvement in ALT will be defined as meeting either of the following conditions:
   ∘ ALT ≤ 40 U/L and ≥ 30% decrease from baseline
   ∘ Liver fat content (MRI-PDFF) ≥ 30% decrease from baseline
- Improvement from baseline in liver fat content measured by MRI PDFF (defined as a ≥ 30% reduction) at Week 48
- Improvement from baseline in ALT (defined as an ALT ≤ 40 U/L with a ≥ 30% decrease from baseline) at Week 48

Table 3 describes the histological scoring system for NAFLD used in this example, as described by D.E. Kleiner et al. for the Nonalcoholic Steatohepatitis Clinical Research Network. Hepatology 41:1313-1321, 2005.

**Table 3. Histological Scoring System for Nonalcoholic Fatty Liver Disease**

| NAS Components | | | |
|---|---|---|---|
| Item | Score | Extent | Definition and Comment |
| | 0 | <5% | Refers to amount of surface area involved by steatosis as evaluated on low to medium power examination. |
| | 1 | 5-33% | |
| | 2 | >33-66% | |
| Steatosis | 3 | >66% | |
| | 0 | No foci | Acidophil bodies are not included in this assessment, nor is portal inflammation |
| | 1 | <2 foci/200x | |
| Lobular | 2 | 2-4 foci/200x | |
| Inflammation | 3 | >4 foci/200x | |
| | 0 | None | |
| | 1 | Few balloon cells | "Few" means rare but definite ballooned hepatocytes as well as cases that are diagnostically borderline |
| Hepatocyte Ballooning | 2 | Many cells/prominent ballooning | Most cases with prominent ballooning also had Mallory's hyalin, but Mallory's hyaline is not scored separately for the NAS |

| Fibrosis Stage (Evaluated separately from NAS) | | | |
|---|---|---|---|
| Item | Score | Extent | Definition and Comment |
| | 0 | None | |
| | 1 | Perisinusoidal or periportal | |
| | 1A | Mild, zone 3, perisinusoidal | "delicate" fibrosis |
| | 1B | Moderate, zone 3, perisinusoidal | "dense" fibrosis |
| | 1C | Portal/periportal | This category is included to accommodate cases with portal and/or peri portal fibrosis without accompanying pericellular/perisinusoidal fibrosis |
| | 2 | Perisinusoidal and portal/periportal | |
| | 3 | Bridging fibrosis | |
| Fibrosis | 4 | Cirrhosis | |
| Scoring interpretation: Total NAS score represents the sum of scores for steatosis, lobular inflammation, and ballooning, and ranges from 0-8. Diagnosis of NASH (or, alternatively, fatty liver not diagnostic of NASH) should be made first, then NAS is used to grade activity. In the reference study, NAS scores of 0-2 occurred in cases largely considered not diagnostic of NASH, scores of 3-4 were evenly divided among those considered not diagnostic, borderline, or positive for NASH. Scores of 5-8 occurred in cases that were largely considered diagnostic of NASH | | | |

### ABBREVIATIONS

MRI-PDFF = magnetic resonance imaging-proton density fat fraction.
MRE = magnetic resonance elastography.
LSM = liver stiffness measurement.
ALT = alanine transaminase.
ALT ULN = 30 IU/L for females and 40 IU/L for males
AST = aspartate aminotransferase.
LDL cholesterol = low-density lipoprotein cholesterol.
HDL cholesterol = High-density lipoprotein cholesterol.
M2BPGi = mac-2 binding protein glycosylation isomer.
ELF test = enhanced liver fibrosis test.
NASH CRN = Nonalcoholic Steatohepatitis Clinical Research Network.
NAS = NAFLD activity score.

### Example 3 (reference):

SGLT2 Inhibitor versus Sulfonylurea on Type 2 Diabetes with NAFLD

### Inclusion Criteria

1. The biopsy consistent with the diagnosis of NAFLD
2. Type 2 diabetes, HbAlc >=7.0%

### Exclusion Criteria

Hepatic virus infections, autoimmune hepatitis, primary biliary cirrhosis, sclerosing cholangitis, haemochromatosis, antitrypsin deficiency, Wilsons disease, history of parenteral nutrition and use of drugs known to induce steatosis or hepatic injury caused by substance abuse and or the current or past consumption of more than 20 g of alcohol daily
Hypersensitivity to or contraindication of glimepiride and tofogliflozin
None type 2 diabetes
Poorly controlled diabetes
Repeated episodes of unexplained hypoglycemia
Concomitant infection or planned surgery
Poorly controlled hypertension
Severe retinopathy
Malignancy on an active therapeutic regimen or malignancy without complete remission or cure Severe health problems not suitable for the study
Pregnant or lactating women
Inability to participate in the study as assessed by the investigators.

### Baseline Characteristics

A total of 40 patients were randomly assigned to receive once-daily tofogliflozin at a dose of 20 mg (20 patients) or to receive once-daily glimepiride (20 patients). All 40 patients (100%) completed the trial. Demographic and baseline clinical characteristics except for gender were similar in both groups. The patients were all Japanese and type 2 diabetes. The mean age was 53.9 years, the mean body weight 82.0 kg, the mean Hemoglobin A1c 8.2%, and the mean NAS 4.45. A total of 18 patients (45%) had stage F1 fibrosis, 11 (27.5%) had stage F2, and 5 (12.5%) had stage F3.

### Participant flow

A total of 40 patients were randomly assigned to receive once-daily tofogliflozin at a dose of 20 mg (20 patients) or to receive once-daily glimepiride (20 patients). All 40 patients (100%) completed the trial. Information for the primary and confirmatory secondary outcomes related to a biopsy at week 48 was available for 39 patients (97.5%). For only one patient with a serious adverse event (2.5%).

### Primary Outcomes

In the glimepiride group, the only histologic improvement from baseline to 48 weeks was a reduction in ballooning necrosis (P=0.025). In contrast, subjects who received tofogliflozin had significant histologic improvements from baseline to 48 weeks in all variables (ratio of the patients with improvement in steatosis, ballooning necrosis, and lobular inflammation were 65, 55, and 50%, respectively). The fibrosis scores improved in the tofogliflozin group (60%, P=0.001 for the comparison of scores before and after treatment), but the change from baseline did not diff er significantly between the tofogliflozin group and the glimepiride group (P=0.172).

### Secondary Outcomes

### Histologic Features

The mean ballooning scores were significantly reduced in both groups. The mean steatosis scores, percentage of steatosis, lobular inflammation score, and fibrosis score were significantly reduced only in the tofogliflozin group. NAS improved significantly compared with baseline values in both groups, and the beneficial effects were greater in the tofogliflozin group than in the glimepiride group (P=0.002).

### Serum Enzyme Levels and Liver Test

There was an early and highly significant decrease in aspartate aminotransferase and alanine aminotransferase levels in the tofogliflozin group. The changes from baseline did not differ significantly between the tofogliflozin group and the glimepiride group. The changes of gamma-glutamyl transferase significantly reduced in the tofogliflozin group (a mean decrease of 23.8 IU/L at week 48, P<0.001 for the comparison with glimepiride). Moreover, the FIB-4 index significantly reduced in the tofogliflozin group, and the effects were greater in the tofogliflozin group than in the glimepiride group.

### Metabolic Parameters

The decrease in the glycemic parameters, such as FPG and HbA1c, were similar. Bodyweight, BMI, and percentage of body fat had a significant reduction in the tofogliflozin group (a mean body weight decrease of 4.8 kg at week 48, P<0.001 for the comparison with glimepiride). These changes occurred in the first 12 weeks and were sustained throughout the period in which the subjects were receiving treatment. In contrast, the changes in CPR, lipid profile, oxidative stress markers, and cytokines were similar in both groups.

### Brief summary

Among patients with biopsy-confirmed NAFLD and type 2 diabetes, a significantly higher percentage of patients had liver histology improvement via beta oxidation and attenuation of inflammation in the liver with tofogliflozin than with glimepiride under similar glucose level reduction. Tofogliflozin had some favorable metabolic markers. SGLT2 inhibitors may have a hepatoprotective effect.

It is intended that the specification and examples be considered as exemplary only; the invention is indicated by the following claims.

## Claims

1. Pemafibrate or a pharmaceutically acceptable salt thereof for use in a method of treating liver stiffness or liver fibrosis in a human patient with noncirrhotic NASH or NAFLD and a NASH CRN fibrosis score of ≥ 1 and < 4 as defined in Table 3, the method comprising administering to the patient a therapeutically effective amount of pemafibrate or the pharmaceutically acceptable salt thereof.

2. Pemafibrate or the pharmaceutically acceptable salt thereof for use in the method of claim 1, the method further comprising administering to the patient a therapeutically effective amount of tofogliflozin or a pharmaceutically acceptable salt thereof.

3. Pemafibrate or the pharmaceutically acceptable salt thereof for use in the method of claim 1 or 2, wherein, at the time of commencing the method, the patient has:
a) a vibration-controlled transient elastography CAP score ≥ 290 dB/m;
b) a LSM (liver stiffness measurement) ≥ 7 kilopascals (kPa) and < 19 kPa; and
c) an AST concentration ≥ 20 U/L.

4. Pemafibrate or the pharmaceutically acceptable salt thereof for use in the method of claim 1 or 2, wherein, at the time of commencing the method, the patient has:
a) a NAS ≥ 4;
b) a NASH CRN steatosis score ≥ 1;
c) a NASH CRN lobular inflammation score ≥ 1; and
d) a NASH CRN ballooning score ≥ 1.

5. Pemafibrate or the pharmaceutically acceptable salt thereof for use in the method of claim 1 or 2, wherein, at the time of commencing the method, the patient has:
a) a NAS ≥ 4;
b) a NASH CRN steatosis score ≥ 1;
c) a NASH CRN lobular inflammation score ≥ 1;
d) a NASH CRN ballooning score ≥ 1; and
e) an ALT concentration ≥ 1X, ≥ 2X, or ≥ 3X of ULN, wherein ALT ULN corresponds to 30 IU/L for females and 40 IU/L for males.

6. Pemafibrate or the pharmaceutically acceptable salt thereof for use in the method of claim 1 or 2, wherein, at the time of commencing the method, the patient has:
a) a hepatic fat fraction on MRI-PDFF (magnetic resonance imaging-proton density fat fraction) ≥ 10%;
b) liver stiffness on MRE (magnetic resonance elastography) ≥ 2.5 kPa; and
c) an ALT level ≥ 1X ULN, wherein ALT ULN corresponds to 30 IU/L for females and 40 IU/L for males.

7. Pemafibrate or the pharmaceutically acceptable salt thereof for use in the method of claim 1 or 2, wherein, at the time of commencing the method, the patient has:
a) a NASH CRN fibrosis score of 1, 2, 3, ≥ 1 and < 4, or ≥ 2 and < 4; and
b) an ALT concentration ≤ 5 × ULN, wherein ALT ULN corresponds to 30 IU/L for females and 40 IU/L for males.

8. Pemafibrate or the pharmaceutically acceptable salt thereof for use in the method of claim 1 or 2, wherein the method comprises:
a) a histological improvement in the patient's NAS of ≥ 2 points; and
b) no worsening of the patient's NASH CRN fibrosis score.

9. Pemafibrate or the pharmaceutically acceptable salt thereof for use in the method of claim 1 or 2, wherein the method comprises:
a) histological resolution of steatohepatitis defined as:
i) absence of fatty liver disease; or
ii) isolated or simple steatosis without steatohepatitis and a NAS of 0-1 for inflammation, 0 for ballooning, and any value for steatosis; and
b) no worsening of NASH CRN fibrosis score.

10. Pemafibrate or the pharmaceutically acceptable salt thereof for use in the method of claim 1 or 2, wherein the method comprises:
a) improvement in the patient's NASH CRN inflammation score ≥ 1;
b) improvement in the patient's NASH CRN ballooning score ≥ 1; and
c) improvement in the patient's NASH CRN steatosis score ≥ 1.

11. Pemafibrate or the pharmaceutically acceptable salt thereof for use in the method of claim 1 or 2, wherein the method comprises:
a) improvement in the patient's NASH CRN fibrosis score ≥ 1;
b) no worsening of the patient's NASH CRN ballooning score;
c) no worsening of the patient's NASH CRN inflammation score; and
d) no worsening of the patient's NASH CRN steatosis score.

12. Pemafibrate or the pharmaceutically acceptable salt thereof for use in the method of claim 1 or 2, wherein the method comprises:
a) a liver fat content decrease as measured by MRI-PDFF (magnetic resonance imaging-proton density fat fraction) of ≥ 30%; and/or
b) a ≥ 30% decrease in ALT to ≤ 40 U/L.

13. Pemafibrate or the pharmaceutically acceptable salt thereof for use in the method of claim 1 or 2, wherein the method comprises administering to the patient orally on a daily basis:
a) from 0.1 to 0.8 mg of pemafibrate or a pharmaceutically acceptable salt thereof;
and optionally
b) from 5 to 60 mg of tofogliflozin or a pharmaceutically acceptable salt thereof.

14. Pemafibrate or the pharmaceutically acceptable salt thereof for use in the method of claim 1 or 2, wherein the method comprises administering to the patient orally on a daily basis:
a) 0.4 mg of pemafibrate or a pharmaceutically acceptable salt thereof; and optionally
b) 20 mg of tofogliflozin or a pharmaceutically acceptable salt thereof.

## Patentansprüche

1. Pemafibrat oder ein pharmazeutisch verträgliches Salz davon zur Verwendung in einem Verfahren von Behandeln von Lebersteifigkeit oder Leberfibrose bei einem humanen Patienten mit nichtzirrhotischer NASH (nichtalkoholischer Steatohepatitis) oder NAFLD (nichtalkoholischer Fettlebererkrankung) und einem NASH CRN (Clinical Research Network) Fibrose-Score von ≥ 1 and < 4 wie in Tabelle 3 definiert, wobei das Verfahren das Verabreichen einer therapeutisch wirksamen Menge von Pemafibrat oder dem pharmazeutisch verträglichen Salz davon an den Patienten umfasst.

2. Pemafibrat oder das pharmazeutisch verträgliche Salz davon zur Verwendung in dem Verfahren nach Anspruch 1, wobei das Verfahren ferner das Verabreichen einer therapeutisch wirksamen Menge von Tofogliflozin oder einem pharmazeutisch verträglichen Salz davon an den Patienten umfasst.

3. Pemafibrat oder das pharmazeutisch verträgliche Salz davon zur Verwendung in dem Verfahren nach Anspruch 1 oder 2, wobei der Patient zum Zeitpunkt des Beginns des Verfahrens aufweist:
a) einen CAP-Score (Controlled-Attenuation-Parameter-Wert) der vibrationskontrollierten transienten Elastographie ≥ 290 dB/m;
b) eine LSM (Lebersteifigkeitsmessung) ≥ 7 Kilopascal (kPa) und < 19 kPa; und
c) eine AST-(Aspartat-Aminotransferase-)Konzentration ≥ 20 U/L.

4. Pemafibrat oder das pharmazeutisch verträgliche Salz davon zur Verwendung in dem Verfahren nach Anspruch 1 oder 2, wobei der Patient zum Zeitpunkt des Beginns des Verfahrens aufweist:
a) einen NAS (NAFLD-Aktivitätsscore) ≥ 4;
b) einen Steatosescore NASH CRN ≥ 1;
c) einen lobulären Entzündungsscore nach NASH CRN ≥ 1; und
d) einen Ballonierungsscore nach NASH CRN ≥ 1.

5. Pemafibrat oder das pharmazeutisch verträgliche Salz davon zur Verwendung in dem Verfahren nach Anspruch 1 oder 2, wobei der Patient zum Zeitpunkt des Beginns des Verfahrens aufweist:
a) einen NAS ≥ 4;
b) einen Steatosescore NASH CRN ≥ 1;
c) einen lobulären Entzündungsscore nach NASH CRN ≥ 1;
d) einen Ballonierungsscore nach NASH CRN ≥ 1; und
e) eine ALT-(Alanin-Aminotransferase-)Konzentration ≥ 1X, ≥ 2X, oder ≥ 3X der ULN (oberen Normgrenze), wobei die ALT ULN (obere Normgrenze der Alanin-Aminotransferase) für Frauen 30 IU/L und für Männer 40 IU/L entspricht.

6. Pemafibrat oder das pharmazeutisch verträgliche Salz davon zur Verwendung in dem Verfahren nach Anspruch 1 oder 2, wobei der Patient zum Zeitpunkt des Beginns des Verfahrens aufweist:
a) einen hepatischen Fettanteil, bestimmt mittels MRT-PDFF (Magnetresonanztomographie-Protonendichte-Fettfraktion) ≥ 10%;
b) Lebersteifigkeit, bestimmt mittels MRE (Magnetresonanzelastographie) ≥ 2,5 kPa;
und
c) eine ALT-Menge ≥ 1X ULN, wobei ALT ULN für Frauen 30 IU/L und für Männer 40 IU/L entspricht.

7. Pemafibrat oder das pharmazeutisch verträgliche Salz davon zur Verwendung in dem Verfahren nach Anspruch 1 oder 2, wobei der Patient zum Zeitpunkt des Beginns des Verfahrens aufweist:
a) einen NASH CRN Fibrose-Score von 1, 2, 3, ≥ 1 und < 4, oder ≥ 2 und < 4; und
b) eine ALT-Konzentration ≤ 5 x ULN, wobei ALT ULN für Frauen 30 IU/L und für Männer 40 IU/L entspricht.

8. Pemafibrat oder das pharmazeutisch verträgliche Salz davon zur Verwendung in dem Verfahren nach Anspruch 1 oder 2, wobei das Verfahren umfasst:
a) eine histologische Verbesserung des NAS des Patienten von ≥ 2 Punkten; und
b) keine Verschlechterung des NASH-CRN-Fibrosescores des Patienten.

9. Pemafibrat oder das pharmazeutisch verträgliche Salz davon zur Verwendung in dem Verfahren nach Anspruch 1 oder 2, wobei das Verfahren umfasst:
a) histologische Auflösung von Steatohepatitis definiert als:
i) Abwesenheit von Fettlebererkrankung; oder
ii) isolierte oder einfache Steatose ohne Steatohepatitis und ein NAS von 0-1 für Entzündung, 0 für Ballonierung, und jedweder Wert für Steatose; und
b) keine Verschlechterung des NASH-CRN-Fibrosescores.

10. Pemafibrat oder das pharmazeutisch verträgliche Salz davon zur Verwendung in dem Verfahren nach Anspruch 1 oder 2, wobei das Verfahren umfasst:
a) Verbesserung des NASH-CRN-Entzündungsscores des Patienten ≥ 1;
b) Verbesserung des NASH-CRN-Ballonierungsscoresdes Patienten ≥ 1; und
c) Verbesserung des NASH-CRN-Steatosescores des Patienten ≥ 1.

11. Pemafibrat oder das pharmazeutisch verträgliche Salz davon zur Verwendung in dem Verfahren nach Anspruch 1 oder 2, wobei das Verfahren umfasst:
a) Verbesserung des NASH CRN Fibrose-Scores des Patienten ≥ 1;
b) keine Verschlechterung des NASH-CRN-Ballonierungsscores des Patienten;
c) keine Verschlechterung des NASH-CRN-Entzündungsscores des Patienten; und
d) keine Verschlechterung des NASH-CRN-Steatosescores des Patienten.

12. Pemafibrat oder das pharmazeutisch verträgliche Salz davon zur Verwendung in dem Verfahren nach Anspruch 1 oder 2, wobei das Verfahren umfasst:
a) eine Fettlebergehalt-Abnahme von ≥ 30%, gemessen mittels MRT-PDFF; und/oder
b) eine ≥ 30%ige Abnahme des ALT-Wertes auf ≤ 40 U/L.

13. Pemafibrat oder das pharmazeutisch verträgliche Salz davon zur Verwendung in dem Verfahren nach Anspruch 1 oder 2, wobei das Verfahren das orale, tägliche Verabreichen folgender Substanzen an den Patienten umfasst::
a) 0,1 bis 0,8 mg Pemafibrat oder eines pharmazeutisch verträglichen Salzes davon;
und gegebenenfalls
b) 5 bis 60 mg Tofogliflozin oder eines pharmazeutisch verträglichen Salzes davon.

14. Pemafibrat oder das pharmazeutisch verträgliche Salz davon zur Verwendung in dem Verfahren nach Anspruch 1 oder 2, wobei das Verfahren das orale, tägliche Verabreichen folgender Substanzen an den Patienten umfasst:
a) 0,4 mg Pemafibrat oder eines pharmazeutisch verträglichen Salzes davon; und gegebenenfalls
b) 20 mg Tofogliflozin oder eines pharmazeutisch verträglichen Salzes davon.

## Revendications

1. Pémafibrate ou un sel pharmaceutiquement acceptable de celui-ci pour utilisation dans une méthode de traitement de la raideur hépatique ou de la fibrose hépatique chez un patient humain atteint de NASH ou de NAFLD non cirrotique et ayant un score de fibrose NASH CRN ≥ 1 et < 4 tel que défini dans le Tableau 3, la méthode comprenant l'administration au patient d'une quantité thérapeutiquement efficace de pémafibrate ou du sel pharmaceutiquement acceptable de celui-ci.

2. Pémafibrate ou le sel pharmaceutiquement acceptable de celui-ci pour utilisation dans la méthode selon la revendication 1, la méthode comprenant en outre l'administration au patient d'une quantité thérapeutiquement efficace de tofogliflozine ou d'un sel pharmaceutiquement acceptable de celle-ci.

3. Pémafibrate ou le sel pharmaceutiquement acceptable de celui-ci pour utilisation dans la méthode selon la revendication 1 ou 2, dans lequel, au moment du début de la méthode, le patient présente :
a) un score CAP d'élastographie transitoire contrôlée par vibration ≥ 290 dB/m ;
b) une LSM (mesure de la raideur hépatique) ≥ 7 kilopascals (kPa) et < 19 kPa ; et
c) une concentration d'AST ≥ 20 U/L.

4. Pémafibrate ou le sel pharmaceutiquement acceptable de celui-ci pour utilisation dans la méthode selon la revendication 1 ou 2, dans lequel, au moment du début de la méthode, le patient presents :
a) un NAS ≥ 4 ;
b) un score de stéatose NASH CRN ≥ 1 ;
c) un score d'inflammation lobulaire NASH CRN > 1 ; et
d) un score de ballonisation NASH CRN ≥ 1.

5. Pémafibrate ou le sel pharmaceutiquement acceptable de celui-ci pour utilisation dans la méthode selon la revendication 1 ou 2, dans lequel, au moment du début de la méthode, le patient présente :
a) un NAS > 4 ;
b) un score de stéatose NASH CRN > 1 ;
c) un score d'inflammation lobulaire NASH CRN ≥ 1 ;
d) un score de ballonisation NASH CRN ≥ 1 ; et
e) une concentration d'ALT ≥ 1X, ≥ 2X, ou ≥ 3X de l'ULN, dans lequel l'ULN de l'ALT correspond à 30 UI/L pour les femmes et 40 UI/L pour les hommes.

6. Pémafibrate ou le sel pharmaceutiquement acceptable de celui-ci pour utilisation dans la méthode selon la revendication 1 ou 2, dans lequel, au moment du début de la méthode, le patient présente :
a) une fraction de graisse hépatique par IRM-PDFF (fraction de graisse de densité protonique par imagerie par résonance magnétique) ≥ 10 % ;
b) une raideur hépatique par MRE (élastographie par résonance magnétique) ≥ 2,5 kPa ; et
c) un taux d'ALT ≥ 1X ULN, dans lequel l'ULN de l'ALT correspond à 30 UI/L pour les femmes et 40 UI/L pour les hommes.

7. Pémafibrate ou le sel pharmaceutiquement acceptable de celui-ci pour utilisation dans la méthode selon la revendication 1 ou 2, dans lequel, au moment du début de la méthode, le patient présente :
a) un score de fibrose NASH CRN de 1, 2, 3, ≥ 1 et < 4, ou ≥ 2 et < 4 ; et
b) une concentration d'ALT ≤ 5×ULN, dans lequel l'ULN de l'ALT correspond à 30 UI/L pour les femmes et 40 UI/L pour les hommes.

8. Pémafibrate ou le sel pharmaceutiquement acceptable de celui-ci pour utilisation dans la méthode selon la revendication 1 ou 2, dans lequel la méthode comprend :
a) une amélioration histologique du NAS du patient ≥ 2 points ; et
b) aucune aggravation du score de fibrose NASH CRN du patient.

9. Pémafibrate ou le sel pharmaceutiquement acceptable de celui-ci pour utilisation dans la méthode selon la revendication 1 ou 2, dans lequel la méthode comprend :
a) la résolution histologique de la stéatohépatite définie comme :
i) l'absence de stéatose hépatique ; ou
ii) une stéatose isolée ou simple sans stéatohépatite et un NAS de 0-1 pour l'inflammation, 0 pour la ballonisation, et n'importe quelle valeur pour la stéatose ; et
b) aucune aggravation du score de fibrose NASH CRN.

10. Pémafibrate ou le sel pharmaceutiquement acceptable de celui-ci pour utilisation dans la méthode selon la revendication 1 ou 2, dans lequel la méthode comprend :
a) une amélioration du score d'inflammation NASH CRN du patient ≥ 1 ;
b) une amélioration du score de ballonisation NASH CRN du patient ≥ 1 ; et
c) une amélioration du score de stéatose NASH CRN du patient ≥ 1.

11. Pémafibrate ou le sel pharmaceutiquement acceptable de celui-ci pour utilisation dans la méthode selon la revendication 1 ou 2, dans lequel la méthode comprend :
a) une amélioration du score de fibrose NASH CRN du patient ≥ 1 ;
b) aucune aggravation du score de ballonisation NASH CRN du patient ;
c) aucune aggravation du score d'inflammation NASH CRN du patient ; et
d) aucune aggravation du score de stéatose NASH CRN du patient.

12. Pémafibrate ou le sel pharmaceutiquement acceptable de celui-ci pour utilisation dans la méthode selon la revendication 1 ou 2, dans lequel la méthode comprend :
a) une diminution de la teneur en graisse hépatique telle que mesurée par IRM-PDFF (fraction de graisse de densité protonique par imagerie par résonance magnétique) ≥ 30 % ; et/ou
b) une diminution ≥ 30 % de l'ALT jusqu'à une valeur ≤ 40 U/L.

13. Pémafibrate ou le sel pharmaceutiquement acceptable de celui-ci pour utilisation dans la méthode selon la revendication 1 ou 2, dans lequel la méthode comprend l'administration au patient par voie orale et sur une base quotidienne :
a) de 0,1 à 0,8 mg de pémafibrate ou d'un sel pharmaceutiquement acceptable de celui-ci ; et facultativement
b) de 5 à 60 mg de tofogliflozine ou d'un sel pharmaceutiquement acceptable de celle-ci.

14. Pémafibrate ou le sel pharmaceutiquement acceptable de celui-ci pour utilisation dans la méthode selon la revendication 1 ou 2, dans lequel la méthode comprend l'administration au patient par voie orale et sur une base quotidienne :
a) de 0,4 mg de pémafibrate ou d'un sel pharmaceutiquement acceptable de celui-ci, et facultativement
b) de 20 mg de tofogliflozine ou d'un sel pharmaceutiquement acceptable de celle-ci.
